# EUROPÄISCHE PATENTSCHRIFT

(11) **EP 2 730 178 B1**
(45) Veröffentlichungstag und Bekanntmachung des Hinweises auf die Patenterteilung: **26.08.2020**
(21) Anmeldenummer: 12192171.2
(22) Anmeldetag: 12.11.2012
(51) Int. Cl.: A23F 3/16, C12G 3/06, A23L 27/00, A23L 27/20, A23L 13/40, A23L 2/56, A61K 8/37, A23L 15/00, A61Q 11/00

(54) **Zubereitungen zur oralen Aufnahme**
Oral compositions
Composition prise par voie orale

(43) Veröffentlichungstag der Anmeldung: 14.05.2014
(73) Patentinhaber: Symrise AG, 37603 Holzminden (DE)
(72) Erfinder: Obst, Katja, 04229 Leipzig (DE); Krammer, Gerhard, 37603 Holzminden (DE); Ley, Jakob, 37603 Holzminden (DE); Reichelt, Katharina, 37603 Holzminden (DE)
(74) Vertreter: Eisenführ Speiser

(56) Entgegenhaltungen:
- WO-A1-94/07477
- DE-A1-102010 049 708
- JP-A- 2004 161 679
- US-A1- 2007 178 123
- US-A1- 2012 196 018
- DATABASE WPI Week 200528 Thomson Scientific, London, GB; AN 2005-266728 & JP 2005 082594 A (BOEHRINGER INGELHEIM INT GMBH) 31 March 2005 (2005-03-31)
- Jakob P. Ley: "Masking Bitter Taste by Molecules", CHEMOSENSORY PERCEPTION, vol. 1, no. 1, 13 February 2008 (2008-02-13), pages 58-77, XP055562140, US ISSN: 1936-5802, DOI: 10.1007/s12078-008-9008-2
- Masataka Narukawa ET AL: "Taste characterisation of green tea catechins : Taste characterisation of catechins", INTERNATIONAL JOURNAL OF FOOD SCIENCE AND TECHNOLOGY., vol. 45, no. 8, 1 August 2010 (2010-08-01) , pages 1579-1585, XP055632356, GB ISSN: 0950-5423, DOI: 10.1111/j.1365-2621.2010.02304.x
- H. Valentová ET AL: "SENSORY EVALUATION | Taste" In: "Encyclopedia of Food Sciences and Nutrition", 1 January 2003 (2003-01-01), Elsevier, XP055632392, ISBN: 978-0-12-227055-0 pages 5180-5187, DOI: 10.1016/B0-12-227055-X/01069-5,

## Beschreibung

### Gebiet der Erfindung

Die Erfindung befindet sich auf dem Gebiet der Aromastoffe und betrifft den Einsatz von speziellen Milchsäureestern zur Maskierung von unangenehmen Geschmacksnoten.

### Stand der Technik

Nahrungs- und Genussmittel enthalten häufig eine Vielzahl verschiedener Bitterstoffe, die zwar einerseits in bestimmten Lebensmitteln in Maßen erwünscht sind und zu deren charakteristischen Geschmack beitragen (z.B. Coffein in Tee oder Kaffee, Chinin in so genannten Bitter-Lemon-Getränken, Bitterstoffe aus Hopfen in Bier), andererseits den Wert aber auch stark mindern können. Dazu gehören z.B. Flavonoidglycoside und Limonoide in Zitrus-Säften, der bittere Nachgeschmack vieler hochintensiver Süßstoffe wie Aspartam, Cyclamat, Acesulfam K, Rebaudiosid A, Glyccyrhizinsäure oder Saccharin sowie der unangenehme Geschmack, den hydrophobe Aminosäuren und Peptide in Käse verursachen können.

Bittergeschmack wird regelmäßig durch einzelne Stoffe verursacht, die an spezielle Bitterrezeptoren auf Geschmackszellen, die in den so genannten Geschmacksknospen auf der Zunge zu finden sind, binden und über neurochemische Kaskaden ein Signal an das Gehirn senden, dass eine Abwehrreaktion und einen negativen Geschmackseindruck verursacht (vgl. Meyerhof, Reviews of Physiology, Biochemistry and Pharmacology 2005, 154, 37-72**).**

Adstringierender Geschmack wird in der Regel durch Fällung von Prolin-reichen Proteinen im Speichel durch Adstringentien, z.B. Metallsalze, Polyphenole wie (Gallo-)Catechine, Proanthocyanidine, andere Flavonoide oder Tannine, verursacht. Der normalerweise als "Schmiermittel" dienende homogene Speichel enthält dann denaturierte Proteine, die die Gleitfähigkeit herabsetzen und dadurch ein raues oder trockenes, auch als adstringierend empfundenes Gefühl im Mund hinterlassen (vgl. Am. J. Clin. Nutr. 2005, 81, 330S-335S**).**

Insbesondere zur Senkung des natürlichen Gehalts an Adstringentien ist dementsprechend oft eine nachträgliche Behandlung nötig, beispielsweise durch einen zu verwerfenden Voraufguss bei grünem Tee zur Entfernung von Catechinen, oder enzymatisch, z.B. Behandlung von Tee mit zersetzenden Enzymen zur Zerstörung der adstringierenden Polyphenole, wie in WO 2003 022065 A1 (Unicafe) oder JP 2007 135481 A1 (Kikkoman) beschrieben, oder Einsatz von speziellen Peptidasen bei der Reifung von Käse.

Diese Behandlungen zur Senkung des natürlichen Gehalts an Adstringentien sind belastend für das Produkt, erzeugen Abfallstoffe und bedingen z.B. auch Lösungsmittelreste und andere Rückstände (Enzyme) in den Produkten.

Daher ist es wünschenswert, Stoffe zu finden, die unangenehme Geschmackseindrücke, insbesondere adstringierende, trockene, mehlige, staubige, kalkige und/oder metallische Geschmackseindrücke, in kleinsten Konzentrationen wirkungsvoll unterdrücken, oder zumindest vermindern können.

Besonders wichtig ist die Unterdrückung unangenehmer Geschmackseindrücke auch bei vielen pharmazeutischen Wirkstoffen, da dadurch die Bereitschaft der Patienten, insbesondere bei Kindern, die den Wirkstoff enthaltende Zubereitung oral zu sich zu nehmen, deutlich erhöht werden kann. Viele pharmazeutische Wirkstoffe, beispielsweise Aspirin, Salicin, Paracetamol, Ambroxol oder Chinin, um nur eine sehr kleine Auswahl zur Verdeutlichung zu nennen, haben einen ausgeprägten adstringierenden und/oder metallischen Geschmack und/oder Nachgeschmack.

Klassischerweise werden als Gegenmittel für Adstringentien Fettemulsionen verwendet, deren Verwendung in vielen Fällen aber nicht angezeigt ist, so z.B. in fettfreien Getränken oder gesundheitsbewussten fettarmen Produkten.

In JP 2000 287630 A1 (Shiga) und WO 2006 013930 A1 (Suntory) wird eine Methode beschrieben, den adstringierenden Geschmack von Polyphenolen in Teegetränken unter Verwendung von Zucker und bestimmten Aminosäuren zu mindern. Dabei werden die Aminosäuren im Bereich von 0,04 bis 0,1 % eingesetzt.

In JP 2001 046037 A1 (Kikkoman) wird die Minderung des adstringierenden Geschmacks von Polyphenolen mit Stärke und Proteinen erreicht. Dieses Verfahren birgt jedoch den Nachteil, dass insbesondere Proteine und Stärken großen Einfluss auf die rheologischen Eigenschaften der Produkte haben.

In JP 2003 128664 A1 (Nagaoka) wird die Reduktion der Adstringenz durch Salzbildung der Polyphenole erreicht. Leider wird dadurch in vielen Fällen die Stabilität dieser Verbindungen gemindert und die Oxidationsanfälligkeit derselben gesteigert.

In JP 2004 315 441 A1 (Taisho) wird beschrieben, dass man den adstringierenden Geschmack von Eisensalzen mit speziellen Aminosäuren wie γ-Aminobuttersäure reduzieren kann. Durch den notwendigen Einsatz extrem hoher Konzentrationen (1 % und mehr) erhält das Produkt bzw. die Zubereitung jedoch einen sauren Geschmack.

Spezielle Zucker wie Palatinose, auch in Kombination mit Süßstoffen, wurden zur Maskierung der Adstringenz von Sojagetränken in der WO 2004 062385 A1 (Mitsui) vorgeschlagen, allerdings sind auch hier sehr große Mengen erforderlich. In WO 2005 016031 A1 (Cargill) werden nichtreduzierende Disaccharide wie Trehalose als Adstringenz-Maskierer vorgeschlagen. In JP 2004 337132 A1 (Mitsui) wird die Maskierung mit Cyclofructanen beschrieben, die die adstringierenden Stoffe durch Komplexierung maskieren. Gemäß JP 2005 145933 A1 (Taiyo) sind größere Konzentrationen (> 0,1 %) Pektin oder Alginate ebenfalls in der Lage, die Adstringenz von Polyphenolen z.B. aus Traubenkernextrakten zu reduzieren. Gallat-Catechine, wie sie z.B. in Grüntee vorkommen, wurden ebenfalls mit dieser Methode maskiert (vgl. *N*. Hayashi et al. in: Biosci. Biotechnol. Biochem. 2005, 69 (7), 1306-1310**).**

In EP 2058297 A1 (Symrise) wird die Verwendung von ungesättigten Alkamiden wie z.B. Pellitorin oder Mischungen solcher ungesättigten aliphatischen Alkamide wie Spilanthol und Pellitorin zur Minderung der adstringierenden Eigenschaften von oral konsumierbaren Zubereitungen beschrieben; dabei ist aber durch die notwendige wirksame Konzentration auch der trigeminale Effekt, insbesondere der als Kribbeln oder "Tingling" beschriebene trigeminale Effekt, noch deutlich ausgeprägt und kann die Anwendung als Adstringenzmaskierung stören.

DE 102010049708 A1 (Hexal) beschreibt die Verwendung von Mischungen enthaltend bestimmte monocyclische Monoterpene (1-(2,6,6-Trimethylcyclohexenyl)-2-buten-1-on, N-Ethyl-2-(isopropyl)-5-methyl-cyclohexancarboxamid, Menthyllactat, Mentholethylenglycolcarbonat und Mentholpropylenglycolcarbonat), anorganische oder organische Salze, sowie einen oder mehrere Süßstoffe zur Maskierung bitter schmeckender Arzneimittel (z.B. Cetrizidin, in einer pharmazeutischen Filmformulierung. Aufgrund der hohen Dosierung der auch als physiologische Kühlwirkung auslösenden Monoterpene von 0,01 - 10% ist jedoch eine starke Kühlwirkung wahrzunehmen, die für den angestrebten Einsatz als Maskierer in Lebensmittel störend wirkt.

Teilweise werden auch Süßstoffe zur Reduktion der Adstringenz eingesetzt, wie z.B. in JP 2007 135481 A1 (Kikkoman) beschrieben. Dabei erhält man aber grundsätzlich nur süße Applikationen.

US 2012/0196018 A1 offenbart ein oral verträgliches Produkt, umfassend einen Bestandteil oder Bestandteile, die in Verbindung mit ihrer Verwendung einen unakzeptablen Nachgeschmack haben, und eine kühlende Verbindung, die als Geschmacksmaskierungsmittel in einer Menge von 0,5 ppm oder weniger verwendet wird.

Bei den oben erwähnten, bekannten Verfahren besteht neben den bereits genannten Nachteilen regelmäßig ein weiterer Nachteil darin, dass die Maskierungsmittel in erheblichen Mengen (> 0,05 %-Bereich) eingesetzt werden sollten, was zu höheren Kosten und im Falle der polymeren Kohlenhydrate oder Proteine auch zu Anwendungsproblemen führt.

Die komplexe Aufgabe, die der vorliegenden Erfindung zugrunde liegt, hat daher darin bestanden, Stoffe zu finden, die gleichzeitig in sehr geringen Mengen zur Maskierung oder Verminderung des unangenehmen Geschmackseindrucks unangenehm schmeckender Stoffe geeignet sind und insbesondere einen Adstringenz-maskierenden Effekt gegen eine Vielzahl von Adstringentien zeigen, breit anwendbar sowie leicht zugänglich sind und im Idealfall natürlich vorkommen.

### Beschreibung der Erfindung

Gegenstand der Erfindung ist die Verwendung von Milchsäure-1-menthylestern der Formel (I) zur Maskierung oder Reduzierung von unangenehmen Geschmackseindrücken bei Zubereitungen zur oralen Aufnahme 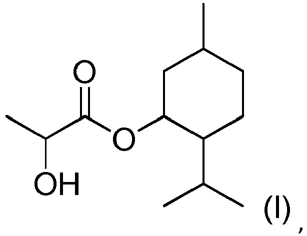 wobei die Konzentration der Milchsäure-1-menthylester der Formel (I) in den Zubereitungen zur oralen Aufnahme unter der Konzentration liegt, bei der die Verbindungen der Formel (I) einen wahrnehmbaren physiologischen Kühleffekt verursachen, wobei es sich bei den unangenehmen Geschmackseindrücken um adstringierend, trocken, staubig, mehlig und/oder kalkig handelt, und wobei die Zubereitungen zur oralen Aufnahme Stoffe umfassen, ausgewählt aus der Gruppe, die gebildet wird von (b1) Catechinen, (b2) Proanthocyanidinen, (b3) Coffein, (b4) Theobromin, (b5) Naringin und (b6) Süßstoffen.

Überraschenderweise wurde gefunden, dass sich sämtliche der obengenannte Milchsäurementhylester besonders gut dazu eignen, einen unangenehmen Geschmack oder Nachgeschmack eines unangenehm schmeckenden, insbesondere eines adstringierenden Stoffes oder Stoffgemisches zu verändern, insbesondere zu maskieren oder zu vermindern. Ebenfalls maskiert bzw. reduziert werden unangenehme Geschmackseindrücke vom Typ trocken, staubig, mehlig, kalkig oder metallisch. Dabei ist besonders überraschend, dass die für die erfindungsgemäße Maskierung notwendige Konzentration der Milchsäurementhylester regelmäßig unter der Konzentration liegt, bei der die Verbindungen der Formel (1) einen wahrnehmbaren physiologischen Kühleffekt verursachen.

### Milchsäure-1-menthylester

Die bevorzugten Milchsäure-l-menthylester und ihre möglichen Stereoisomere sind in der folgenden Abbildung als Formeln (1-LL), (1-LD), (1-DL), und (1-DD) noch einmal zur Verdeutlichung aufgeführt:

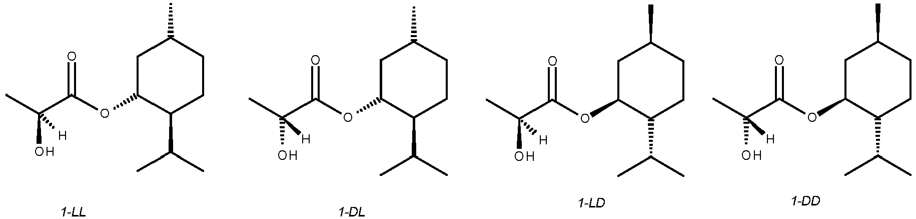

Besonders bevorzugt sind dabei L-Milchsäure-L-menthylester der Formel (1-LL) und D-Milchsäure-L-menthylester der Formel (1-DL) als Einzelverbindungen in einer beliebigen Mischung, besonders bevorzugt in einer Mischung mit mehr als 50 Gew.-%, insbesondere bevorzugt mehr als 75 Gew.-%, ganz besonders bevorzugt mehr als 95 Gew.-% L-Milchsäure-L-menthylester der Formel (1-LL) bezogen auf die Gesamtmasse an Milchsäurementhylestern der Formel (1).

Diese besonders bevorzugten Mischungen der Milchsäurementhylester der Formeln (1-LL) und (1-DL) sind auch in der Natur zu finden (vgl. Gassenmeier, Flavour and Fragrance Journal 2006, 21, (4), 725-730**).**

Daher sind insbesondere natürlich vorkommende oder durch physikalische Trennprozesse isolierte oder über enzymatische, fermentative oder übliche Prozesse der Lebensmittelzubereitung natürlich hergestellte oben definierte Mischungen der Milchsäurementhylester der Formeln (1-LL) und (1-DL) bevorzugt; diese Mischungen können aus ätherischen Ölen der Mentha-Familie, ggfs. nach Fermentation mit Milchsäurebakterien oder durch natürliche Veresterung von natürlicher Milchsäure mit natürlichem L-Menthol gewonnen werden.

### Stoffe mit unangenehmen Geschmackseindrücken

Die vorgenannten unangenehm schmeckenden Stoffe, die die Komponente (b) bilden, können weitere, nicht unangenehme Geschmacks- und/oder Geruchsqualitäten besitzen. Im Rahmen dieses Textes sind als nicht unangenehme Geschmacksqualitäten bevorzugt die Eindrücke würzig, umami, süß, salzig, sauer, scharf, kühlend, wärmend, brennend oder kribbelnd zu nennen.

Beispiele für Stoffe mit unangenehmen Geschmackseigenschaften, die die Komponente (b) bilden, sind beispielsweise die Folgenden:
- Catechine und Proanthocyanidine in einer Gesamtmenge von beispielsweise zumindest 0,01 Gew.-%, vorzugsweise von zumindest 0,05 Gew.-%, bevorzugt im Bereich von 0,075 Gew.-% bis 1 Gew.-%,
- Coffein und Theobromin in einer Gesamtmenge von beispielsweise zumindest 0,005 Gew.-%, vorzugsweise von zumindest 0,01 Gew.-%, bevorzugt von zumindest 0,02 Gew.-%, weiter bevorzugt im Bereich von 0,025 Gew.-% bis 1 Gew.-%,
- Naringin in einer Konzentration von beispielsweise zumindest 0,005 Gew.-%, vorzugsweise von zumindest 0,01 Gew.-%, bevorzugt im Bereich von 0,02 Gew.-% bis 0,5 Gew.-%,
- Süßstoffe in einer Gesamtmenge von beispielsweise zumindest 0,005 Gew.-%, vorzugsweise von zumindest 0,05 Gew.-%, bevorzugt im Bereich von 0,1 Gew.-% bis 2 Gew.-%,
jeweils bezogen auf das Gesamtgewicht der Zubereitung.

Stoffe, die bitter, adstringierend, pappig, kalkig, staubig, trocken, mehlig, ranzig und/oder metallisch schmecken, sind beispielsweise: Xanthinalkaloide, Xanthine (Coffein, Theobromin, Theophyllin), Alkaloide (Chinin, Brucin, Strychnin, Nicotin), phenolische Glycoside (z.B. Salicin, Arbutin), Flavanoidglycoside (z.B. Neohesperidin, Eriocitron, Neoeriocitrin, Narirutin, Hesperidin, Naringin), Chalcone oder Chalconglycoside, Dihydrochalconglycoside (Phloridzin, Trilobatin), hydrolisierbare Tannine (Gallus- oder Elagsäureester von Kohlenhydraten, z.B. Pentagalloylglucose), nichthydrolisierbare Tannine (ggfs. galloylierte Catechine oder Epicatechine und deren Oligomeren, z.B. Proanthyocyanidine oder Procyanidine, Thearubigenin), Flavone und deren Glycoside (z.B. Quercetin, Quercitrin, Rutin, Taxifolin, Myricetin, Myrictrin), andere Polyphenole (γ-Oryzanol, Kaffeesäure oder deren Ester), terpenoide Bitterstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (Kaliumchlorid, Natriumsulfat, Magnesiumsalze, Eisensalze, Aluminiumsalze, Zinksalze), pharmazeutische Wirkstoffe (z.B. Fluorchinolon-Antibiotika, Paracetamol, Aspirin, β-Lactam-Antibiotika, Ambroxol, Propylthiouracil [PROP], Guaifenesin), Vitamine (beispielsweise Vitamin H, Vitamine aus der B-Reihe wie Vitamin B1, B2, B6, B12, Niacin, Panthotensäure), Denatoniumbenzoat oder andere Denatoniumsalze, Sucraloseoctaacetat, Harnstoff, ungesättigte Fettsäuren, insbesondere ungesättigte Fettsäuren in Emulsionen, Aminosäuren (z.B. Leucin, Isoleucin, Valin, Tryptophan, Prolin, Histidin, Tyrosin, Lysin oder Phenylalanin), Peptide (insbesondere Peptide mit einer Aminosäure aus der Gruppe Leucin, Isoleucin, Valin, Tryptophan, Prolin oder Phenylalanin am N- oder C-Terminus). Die vorgenannten Stoffe können entweder einzeln oder als Gemisch vorkommen, bevorzugt auch als natürliche Extrakte aus frischen, getrockneten, gerösteten, und/oder fermentierten Pflanzen oder Pflanzenteilen, so z.B. als Extrakte aus Blättern, Früchten, Ästen, Wurzeln, Fruchtschalen, Kernen, Samen z.B. aus Camellia sinensis, Camellia japonica, Coffea ssp., Cocoa theobroma, Vitis vinifera, Citrus ssp. und Hybriden, Poncirus ssp. und Hybriden, Perilla, Humulus lupulus, oder verwandten Arten stammen.

Erfindungsgemäß zu maskierende Bitterstoffe sind dabei insbesondere Xanthine (insbesondere Coffein, Theobromin, Theophyllin), phenolische Glycoside (insbesondere Salicin, Arbutin), Flavanoidglycoside (insbesondere Neohesperedin, Eriocitrin, Neoeriocitrin, Narirutin, Hesperidin, Naringin), Chalcone oder Chalconglycoside, Dihydrochalconglycoside (insbesondere Phloridzin, Trilobatin), hydrolisierbare Tannine (insbesondere Gallus- oder Ellagsäureester von Kohlenhydraten, z.B. Pentagalloylglucose), nichthydrolisierbare Tannine (insbesondere galloylierte Catechine oder Epicatechine und deren Oligomeren, z.B. Proanthyocyanidine oder Procyanidine, Thearubigenin), Flavone und deren Glycoside (insbesondere Quercetin, Quercitrin, Rutin, Taxifolin, Myricetin, Myrictrin), Kaffeesäure oder deren Ester, terpenoide Bitterstoffe (insbesondere Limonin, Nomilin, Lupolone und Humolone), metallische Salze (Kaliumchlorid, Natriumsulfat, Magnesiumsalze, Eisensalze, Aluminiumsalze, Zinksalze), pharmazeutische Wirkstoffe (z.B. Fluorchinolon-Antibiotika, Paracetamol, Aspirin, β-Lactam-Antibiotika, Ambroxol, Propylthiouracil [PROP], Guaifenesin), Aminosäuren (z.B. Leucin, Isoleucin, Valin, Tryptophan, Prolin, Histidin, Tyrosin, Lysin oder Phenylalanin), Peptide (insbesondere Peptide mit einer Aminosäure aus der Gruppe Leucin, Isoleucin, Valin, Tryptophan, Prolin oder Phenylalanin am N- oder C-Terminus).

Speziell bevorzugte zu maskierende Bitterstoffe sind ausgewählt aus der Gruppe bestehend aus Coffein, Theobromin, Chinin, Salicin, Arbutin, Neohesperedin, Eriocitrin, Neoeriocitrin, Narirutin, Hesperidin, Naringin, Phloridzin, Catechin, Epicatechin, Epigallocatechingallat (EGCG), Gallocatechin, Gallocatechin-3-gallat, Procyanidin B2, Procyanidin B5, Procyanidin C1, Thearubigenin, Rutin, Taxifolin, Myricetin, Myrictrin, Kaffeesäure oder deren Ester, Limonin und Nomilin, Aminosäuren (z.B. Leucin, Isoleucin, Valin, Tryptophan, Prolin, Histidin, Tyrosin, Lysin oder Phenylalanin), Peptide mit einer Aminosäure aus der Gruppe Leucin, Isoleucin, Valin, Tryptophan, Prolin oder Phenylalanin am N- oder C-Terminus, Kaliumchlorid, Paracetamol, Aspirin und β-Lactam-Antibiotika.

Stoffe, die einen bitteren, adstringierenden, pappigen, kalkigen, staubigen, trockenen, mehligen, ranzigen und/oder metallischen Neben- und/oder Nachgeschmack haben, können Aroma- oder Geschmackstoffe mit einem nicht unangenehmen Primärgeschmack (beispielsweise süß, salzig, würzig, sauer) und/oder -geruch sein, und z.B. zur Gruppe der Süßstoffe, Zuckeraustauschstoffe oder der Aromastoffe gehören. Beispielsweise seien genannt: Aspartam, Neotam, Superaspartam, Alitam, Saccharin, Sucralose, Tagatose, Monellin, Monatin, Stevioside, Rubusosid, Steviosid, Rebaudiosid A, Rebaudioside C, Thaumatin, Miraculin, Glycyrrhizin (Glycyrrhizinsäure), Glycyrrhetinsäure oder deren Derivate, Cyclamat oder die physiologisch akzeptablen Salze der vorgenannten Verbindungen

Da sich die Bitterintensität verschiedener Bitterstoffe deutlich unterscheidet, wird die Bitterkeit einer Verbindung im Folgenden manchmal in relativen Bitterequivalenten (RBE) angegeben. Als Bezugssubstanz wird hier der bekannte Bitterstoff Coffein verwendet. Die Bestimmung des RBE-Wertes als Maß für die relative Bitterkeit einer Probe erfolgt mit Hilfe einer Skala von 1 bis 10. Eine relative Bitterkeit von 1, d.h. eine RBE-Wert von 1, entspricht dabei der Bitterkeit einer Menge von Coffein in der Dosierung von 100 mg/kg zu untersuchender Probe. Eine relative Bitterkeit von 5, d.h. eine RBE-Wert von 5, entspricht dabei der Bitterkeit einer Menge von Coffein in der Dosierung von 500 mg/kg zu untersuchender Probe. Die zu untersuchende Probe kann dabei in ihrer Zusammensetzung stark variieren. So kann es sich bei der zu untersuchenden Probe beispielsweise um eine der Ernährung, der Mundpflege, dem Genuss dienende Zubereitung, eine orale pharmazeutischen Zubereitung oder eine kosmetischen Zubereitung, beispielsweise um ein Lebensmittel, ein Getränk, ein Kaugummi, ein Mundwasser, ein Bonbon, einen Hustensaft oder eine Tablette handeln. Die verwendete Skala zur Bestimmung der RBE-Werte entspricht ISO 4121 [Sensory Analysis - Guidelines for the use of quantitative response scales; A.3 Beispiel 2].Die Auswahl der Panelisten zur Bestimmung der RBE-Werte erfolgt gemäß ISO 8586-1 [Sensory analysis - General guidance for the selection, training, and monitoring,of assessors - Part 1: Selected assessors]. Die Zahl der Panelisten entspricht dabei ISO 8586-1, 4.2.3 [Number of persons to be selected, zusammen mit ISO 6658 Sensory analysis - Methodology - General guidance - 5.3.5 Scoring (5 oder mehr ausgewählte Panelisten)]. Dabei besteht der entscheidende Vorteil der erfindungsgemäß einzusetzenden Milchsäurementhylester darin, dass auch solche Bitterstoffe in ihrem unangenehmen Geschmack kompensiert werden können, wenn sie in einer Konzentration vorliegen, die mindestens 2 relativen Bitterequivalenten entspricht.

### Aromastoffe

Die erfindungsgemäß zu verwendenden oralen Zubereitungen können als fakultative Komponente (c) einen oder mehrere Aromastoffe enthalten.

Überraschenderweise wurde des Weiteren gefunden, dass die erfindungsgemäß zu verwendenden Milchsäurementhylester in Kombination mit einem oder mehreren Aromastoffen, vorzugsweise Aromastoffen, die einen milchig-sahnigen und Mundfülle gebenden und/oder einen süß-karamelligen Geschmackseindruck vermitteln, besonders gut den unangenehmen Geschmack eines wie oben definierten unangenehm schmeckenden Stoffes verändern, insbesondere maskieren oder vermindern. Diese Kombinationen sind demnach als weitere erfindungsgemäße Mischungen anzusehen. Dementsprechend betrifft ein Aspekt der vorliegenden Erfindung die Verwendung der Milchsäurementhylester, in Mischung mit einem oder mehreren Aromastoffen, die einen milchig-sahnigen und Mundfülle gebenden und/oder einen süß-karamelligen Geschmackseindruck vermitteln.

Entsprechend sind auch hier erfindungsgemäße Verwendungen in einer zur oralen Aufnahme bestimmten pharmazeutischen oder einer der Ernährung (insbesondere auf Grün- oder Schwarztee-basierende Lebensmittel, auf Catechin- und/oder Tannin-reichen, adstringierend schmeckenden Pflanzenteilen (z.B. Früchte bestimmter Rosacea-Arten, bestimmte Apfelarten, Weintrauben, Wein, Traubenkernextrakten, Rhabarber, Amla, Kakao, Mate) beruhende Produkte, auf Soja basierende Produkte und fettreduzierte (low-fat) Milchprodukte, insbesondere Joghurts), der Mundpflege oder dem Genuss dienenden Zubereitung bevorzugt.

Bei den erfindungsgemäßen Verwendungen einer Mischung, die einen oder mehrere Aromastoffe umfasst, ist eine Gesamtmenge an dem oder den erfindungsgemäß zu verwendenden Milchsäurementhylester besonders geeignet, die nicht ausreicht, den Speichelfluss zu verstärken.

Besonders überraschend ist, dass die in den erfindungsgemäßen Verwendungen bevorzugt eingesetzten Aromastoffe die positive Eigenschaft aufweisen, die Wirkung der Milchsäurementhylester den unangenehmen Geschmackseindruck zu verändern, insbesondere zu maskieren oder zu vermindern in synergistischer Art und Weise zu verstärken. Dementsprechend besteht ein weiterer Aspekt der vorliegenden Erfindung in einer wie weiter oben definierten Verwendung, wobei die eingesetzte Menge an dem oder den Aromastoffen die den unangenehmen Geschmackseindruck eines unangenehm schmeckenden Stoffes oder Stoffgemisches verändernde, insbesondere maskierende oder vermindernde Wirkung des oder der Milchsäurementhylester synergistisch verstärkt.

Eine solche synergistische Verstärkung tritt insbesondere auf, wenn das Gewichtsverhältnis der Menge von erfindungsgemäß zu verwendenden Milchsäurementhylestern und Aromastoffen im Bereich von etwa 1:2 bis etwa 1:200 liegt, vorzugsweise im Bereich von etwa 1:3 bis etwa 1:100, besonders bevorzugt im Bereich von etwa 1:5 bis etwa 1:70. Entsprechende Mischungen sind bevorzugt.

In den durchgeführten Untersuchungen hat sich zudem herausgestellt, dass bei den erfindungsgemäßen Verwendungen von Mischungen, die wie weiter oben definiert einen oder mehrere Aromastoffe enthalten, der eine oder die mehreren Aromastoffe, vorzugsweise ausgewählt sind aus der Gruppe bestehend aus Alkohole, Aldehyde, Ketone, organische Säuren, Ester, Lactone, Schwefelkomponenten, Acetale, Phenole, Furane, Pyrane und Pyrazine.

Typische Beispiele umfassen: Acetophenon, Allylcapronat, alpha-lonon, beta-lonon, Anisaldehyd, Anisylacetat, Anisylformiat, Benzaldehyd, Benzothiazol, Benzylacetat, Benzylalkohol, Benzylbenzoat, beta-lonon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-lsobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Besonders bevorzugt sind die folgenden Aromastoffe, da sie einen milchig-sahnigen und Mundfülle gebenden und/oder einen süß-karamelligen Geschmackseindruck vermitteln: Diacetyl, Acetoin, Benzaldehyd, Furaneol, Heliotropin, Vanillin, Ethylvanillin, Ethylvanillinisobutyrat (= 3-Ethoxy-4-isobutyryloxybenzaldehyd), Furaneol® (2,5-Dimethyl-4-hydroxy-3(2H)-furanon) und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalalcton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-l-al und Phenylacetaldehyd.

### Konfektionierte Produkte

Unter oralen Zubereitungen, die Gegenstand der erfindungsgemäßen Verwendung sind, werden im Folgenden nicht nur solche Produkte verstanden, die der menschlichen Ernährung dienen, sondern auch solche Mittel, die mit der Mundschleimhaut in Kontakt kommen. Somit umfasst diese Bezeichnung Nahrungsmittel auf der einen und Mund- und Zahnreinigungs- und -pflegemittel sowie oral verabreichte pharmazeutische Mittel auf der anderen Seite.

Vorzugsweise handelt es sich bei den oralen Zubereitung um Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, mit (Grün-, Schwarz-)Tee-Extrakten angereicherte (Grün-, Schwarz-)Tee-Getränke, Roibos-Tee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Alternativ kommen Zahnpasten, Mundwässer, Erkältungsmittel oder Wirkstoffkapseln in Betracht.

Die oral konsumierbaren Produkte im Sinne der Erfindung können auch als Halbfertigware zur Herstellung weiterer oral konsumierbarer Produkte dienen. Die oral konsumierbaren süß schmeckenden Produkte im Sinne der Erfindung können auch in Form von Kapseln, Tabletten (nichtüberzogene sowie überzogene Tabletten, beispielsweise magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Die in den oralen Zubereitungen eingesetzte Gesamtmenge der Komponenten (a) und (b) kann bezogen auf die konfektionierten Endprodukte etwa 0,001 bis etwa 1 und vorzugsweise etwa 0,01 bis 0,5 Gew.-% betragen. Insbesondere kann die Menge der Milchsäurementhylester bezogen auf die Halbfertigwaren oder die konfektionierten Endprodukte im Bereich von 0,05 mg/kg (entsprechend 50 ppb) bis 1 g/kg, insbesondere 0,1 mg/kg (entsprechend 0,1 ppm) bis 900 mg/kg vorzugsweise im Bereich von 0,5 bis 750 mg/kg (entsprechend 0,5 bis 900 ppm), bevorzugt im Bereich von 1 bis 500 mg/kg, weiter bevorzugt im Bereich von 3 bis 300 mg/kg, besonders bevorzugt im Bereich von 5 bis 200 mg/kg, am meisten bevorzugt im Bereich von 10 bis 100 mg/kg liegen.

### A. Wirkstoffe zur Maskierung von unangenehmen Geschmackseindrücken

Weiterhin können die oralen Zubereitungen auch weitere Stoffe umfassen, die ebenfalls zur Maskierung von bitteren und/oder adstringierenden Geschmackseindrücken dienen. Diese weiteren Geschmackskorrigenzien werden z. B. aus der folgenden Liste ausgewählt: Nucleotiden (z.B. Adenosin-5'-monophosphat, Cytidin-5'-monophosphat) oder deren physiologisch akzeptablen Salzen, Lactisolen, Natriumsalzen (z.B. Natriumchlorid, Natriumlactat, Natriumcitrat, Natriumacetat, Natriumgluconoat), Hydroxyflavanonen, dabei bevorzugt Eriodictyol, Sterubin (Eriodictyol-7-methylether), Homoeriodictyol, und deren Natrium-, Kalium-, Calcium-, Magnesium- oder Zinksalzen (insbesondere solche wie beschrieben in EP 1258200 A2, die bezüglich der darin offenbarten entsprechenden Verbindungen auf dem Wege der Verweisung Bestandteil dieser Anmeldung wird), Hydroxybenzoesäureamiden, dabei vorzugsweise 2,4-Dihydroxybenzoesäurevanillylamid, 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N*-(4-hydroxy-3-methoxybenzyl)amid, 2,4-Dihydroxybenzoesäure-*N*-(4-hydroxy-3-methoxy-benzyl)amid-Mono-natriumsalz, 2,4-Dihydroxybenzoesäure-*N*-2-(4-hydroxy-3-methoxy-phenyl)-ethylamid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamiden (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, dabei vorzugsweise 2-(4-Hydroxy-3-methoxyphenyl)-1-(2,4,6-trihydroxy-phenyl)ethanon, 1-(2,4-Dihydroxyphenyl)-2-(4-hydroxy-3-methoxyphenyl)-ethanon und 1-(2-Hydroxy-4-methoxyphenyl)-2-(4-hydroxy-3-methoxyphenyl)ethanon) (insbesondere solche wie beschrieben in WO 2006/106023 beschrieben), Hydroxyphenylalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, Diacetyltrimeren (insbesondere solche wie beschrieben in WO 2006/058893); gamma-Aminobuttersäuren (insbesondere solche wie beschrieben in WO 2005/096841); Divanillinen (insbesondere solche wie beschrieben in WO 2004/078302) und 4-Hydroxydihydrochalconen (vorzugsweise wie beschrieben in US 2008/0227867 A1), dabei insbesondere Phloretin und Davidigenin, Aminosäuren oder Gemische von Molkeproteinen mit Lecithinen, Hesperetin wie in der WO 2007/014879 offenbart, 4-Hydroxydihydro-chalko-nen wie in der WO 2007/107596 offenbart, oder Propenylphenylglycosiden (Chavicolglycosiden) wie in EP 1955601 A1 beschrieben, oder Extrakten aus *Rubus suavissimus,* Extrakte aus *Hydrangea macrophylla* wie in EP 2298084 A1 beschrieben, Pellitorin und abgeleiteten Aromakompositionen wie in EP 2008530 A1 beschrieben, Umami-Verbindungen wie in WO 2008/046895 A1 und EP 1989944 A1 beschrieben, Umami-Verbindungen wie beschrieben in EP 2064959 A1 bzw. EP 2135516 A1, Vanillyllignanen, Enterodiol, sowie N-Decadienoyl-aminosäuren und deren Gemische.

### B. Süßstoffe

Die oralen Zubereitungen können insbesondere auch Süßstoffe enthalten, die beispielsweise ausgewählt sind aus den folgenden Gruppen:
- Kohlenhydrate bzw. Zucker ausgewählt aus der Untergruppe bestehend aus Sucrose, D-Fructose, D-Glucose und hochangereicherte Fructose-Sirupe aus Maisstärke (High Fructose Corn Sirup);
- Süßstoffe ausgewählt aus der Untergruppe bestehend aus Steviosid, Rebaudiosid A und Rubusosid, wobei auch Extrakte oder angereicherte Fraktionen dieser Extrakte verwendet werden können, z.B. *Stevia*-Extrakte und *Rubus suavissimus-Extrakte;*
- natürlich vorkommende Süßstoffe, ausgewählt aus der Untergruppe bestehend aus Miraculin, Curculin, Monellin, Mabinlin, Thaumatin, Curculin, Brazzein, Pentadin, D-Phenylalanin, D-Tryptophan und deren Gemische;
- natürlich vorkommende Süßstoffe, ausgewählt aus der Untergruppe bestehend aus Steviosid, Steviolbiosid, Rebaudiosid A, weiteren Steviolglycosiden wie Rebaudiosid B, Rebaudiosid C, Rebaudiosid D, Rebaudiosid E, Rebaudiosid F, Rebaudiosid G, Rebaudiosid H, Dulcosid und/oder Rubusosid, Oslandin, Polypodosid A, Strogin 1, Strogin 2, Strogin 4, Selligueanin A, Dihydroquercetin-3-acetat, Perillartin, Telosmosid A₁₅, Periandrin I-V, Phyllodulcin, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Hernandulcin, Glycyrrhetinsäure, Balansin A, Balansin B und deren Gemiscche;
- süßverstärkende Aroma- und/oder Geschmackstoffe einschließlich deren physiologisch verträglichen Salze, ausgewählt aus der Gruppe bestehend aus Hesperetin, Phloretin, Phyllodulcin oder Extrakte enthaltend Phyllodulcin, Balansin A und/oder Balansin B oder Extrakte aus Mycetia balansae, enthaltend Balansin A und/oder Balansin B, 3',7-Dihydroxy-4'-methoxyflavan, (S)-3',7-Dihydroxy-4'-methoxy-flavan, 1-(2,4-Dihydroxy-phenyl)-3-(3-hydroxy-4-methoxy-phenyl)-propan-1-on, und deren Gemische.

### C. Geschmacksverstärker und Aromastoffe

Die Zubereitungen können des Weiteren zusätzliche Aromastoffe zum Herbeiführen oder Verstärken eines salzigen, gegebenenfalls leicht sauren und/oder Umami-Geschmackseindrucks enthalten. Hierbei bevorzugt sind salzig schmeckende Verbindungen und salzverstärkende Verbindungen. Bevorzugte Verbindungen sind in der WO 2007/045566 A1 offenbart. Ferner bevorzugt sind Umami-Verbindungen wie in der WO 2008/046895 A1 und EP 1989944 A1 beschrieben sind.

Bevorzugte Aromastoffe sind solche, die einen süßen Geruchseindruck verursachen, wobei der oder die weiteren Aromastoffe, die einen süßen Geruchseindruck verursachen, bevorzugt ausgewählt sind aus der Gruppe bestehend aus:
Vanillin, Ethylvanillin, Ethylvanillinisobutyrat (= 3-Ethoxy-4-isobutyryloxybenzaldehyd), Furaneol® (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-l-al, 4-Hydroxyzimtsäure, 4-Methoxy-3-hydroxyzimtsäure, 3-Methoxy-4-hydroxyzimtsäure, 2-Hydroxyzimtsäure, 2,4-Dihydroxybenzoesäure, 3-Hydroxybenzoesäure, 3,4-Dihydroxybenzoesäure, Vanillinsäure, Homovanillinsäure, Vanillomandelsäure und Phenylacetaldehyd.

### D. Weitere Hilfs- und Zusatzstoffe

Beispiele üblicher Grund-, Hilfs- und Zusatzstoffe sind Wasser, Gemische frischer oder prozessierter, pflanzlicher oder tierischer Grund- oder Rohstoffe (beispielsweise rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (beispielsweise Dextrine, Amylose, Amylopektin, Inulin, Xylane, Cellulose), natürliche oder gehärtete Fette (beispielsweise Talg, Schmalz, Palmfett, Kokosfett, gehärtetes Pflanzenfett), Öle (beispielsweise Sonnenblumenöl, Erdnussöl, Maiskeimöl, Olivenöl, Fischöl, Sojaöl, Sesamöl), Fettsäuren oder deren Salze (beispielsweise Kaliumstearat), proteinogene oder nicht-proteinogene Aminosäuren und verwandte Verbindungen (beispielsweise Taurin), Peptide, native oder prozessierte Proteine (beispielsweise Gelatine), Enzyme (beispielsweise Peptidasen), Nukleinsäuren, Nucleotide, andere als die vorbeschriebenen Geschmackskorrigenzien für unangenehme Geschmackseindrücke, Geschmackskorrigenzien für weitere, in der Regel nicht unangenehme Geschmackseindrücke, geschmacksmodulierende Stoffe (beispielsweise Inositolphosphat, Nucleotide wie Guanosinmonophosphat, Adenosinmonophosphat oder andere Stoffe wie Natriumglutamat oder 2-Phenoxypropionsäure), Emulgatoren (beispielsweise Lecithine, Diacylglycerole), Stabilisatoren (beispielsweise Carageenan, Alginat), Konservierungsstoffe (beispielsweise Benzoesäure, Sorbinsäure), Antioxidantien (beispielsweise Tocopherol, Ascorbinsäure), Chelatoren (beispielsweise Citronensäure), organische oder anorganische Säuerungsmittel (beispielsweise Äpfelsäure, Essigsäure, Citronensäure, Weinsäure, Phosphorsäure), zusätzliche Bitterstoffe (beispielsweise Chinin, Coffein, Limonin, Amarogentin, Humolone, Lupolone, Catechine, Tannine), mineralische Salze (beispielsweise Natriumchlorid, Kaliumchlorid, Magnesiumchlorid, Natriumphosphate), die enzymatische Bräunung verhindernde Stoffe (beispielsweise Sulfit, Ascorbinsäure), ätherische Öle, Pflanzenextrakte, natürliche oder synthetische Farbstoffe oder Farbpigmente (beispielsweise Carotinoide, Flavonoide, Anthocyane, Chlorophyll und deren Derivate), Gewürze, synthetische, natürliche oder naturidentische Aromastoffe oder Riechstoffe sowie Geruchskorrigenzien.

### E. Getränke

Als orale Zubereitungen, die die erfindungsgemäßen einzusetzenden Milchsäuremethylester enthalten können, sind insbesondere Getränke zu nennen, die auch karbonisiert sein können. Bevorzugte erfindungsgemäße Getränke sind gegebenenfalls karbonisierte, säure- und fruchthaltige Eistees, gegebenenfalls angereichert mit Catechinen oder Pflanzenextrakten, Limonaden (beispielsweise Orangen-, Limonen- oder Zitronentyp), karbonisierte isotonische Getränke (beispielsweise Orangen-, Limonen oder Zitronentyp), karbonisierte, saure Erfrischungsgetränke (beispielsweise Cola-, Zitrone-, Orange-, Limone-, Kirsch-, Apfel-, Vanille-Typ oder deren Mischung), karbonisierte Schorlen, karbonisierte Obst- und Gemüsesäfte, karbonisierte Frucht- oder Gemüsesaftzubereitungen. Dabei bedeutet karbonisiert im Sinne der Erfindung, dass das Getränk natürlich (z.B. aus Gärungsprozessen wie bei der Bierherstellung oder durch Wasser aus Kohlendioxid-haltigen Mineralquellen) eingetragenes Kohlendioxid enthält oder diesem Kohlendioxid während des Herstell- und/oder Abfüllprozesses zugesetzt wurde.

Diese können als bevorzugte Hilfs- oder Trägerstoffe Maltodextrin, Stärke, natürliche oder künstliche Polysaccharide und/oder Pflanzengummen wie modifizierte Stärken oder Gummi Arabicum, für die Aromamischungen zugelassene Lösungsmittel wie beispielsweise Ethanol, 1,2-Propylenglycol, Wasser, Glycerin, Triacetin, Pflanzenöltriglyceride, färbende Mittel, beispielsweise zugelassene Lebensmittelfarbstoffe, färbende Pflanzenextrakte, Stabilisitoren, Konservierungsstoffe, Antioxidantien und viskositäte-beeinflussende Stoffe enthalten.

Besonders bevorzugte oral konsumierbare süß schmeckende Produkte im Sinne der Erfindung sind alkoholische Getränke wie Biermixgetränke, Weinmixgetränke oder sonstige, maximal 5 Vol.-% Alkohol enthaltende Mischgetränke und/oder nichtalkoholische Getränke wie Tee, Eistee (gesüßt, beispielsweise auch mit Kräuteraromen oder Fruchtaromen vom Typ Zitrone, Orange), (carbonisierte) fruchthaltige Limonaden (beispielsweise Orangen-, Limonen- oder Zitronentyp), (carbonisierte) isotonische Getränke (beispielsweise Orangen-, Limonen oder Zitronentyp), (carbonisierte) Erfrischungsgetränke (beispielsweise Cola-, Zitrone-, Orange-, Limone-, Kirsch-, Apfel-, Vanille-Typ oder deren Mischung), Nektare, Schorlen, Milchgetränke, Buttermilchgetränke, Joghurt, Kefir, Molkegetränke, Sojamilch und daraus gefertigte Produkte, Fruchtgetränke mit Sojaprotein, Haferprotein-Getränke, und Instantgetränke (beispielsweise Instant-Kakao-Getränke, Instant-Tee-Getränke, Instant-Kaffee-getränke, Instant-Fruchtgetränke), sogenannte aromatisierte Wässer ("Near Water"-Getränke, wobei letztere eine Süßung haben müssen.

"Near Water"-Getränke im Sinne dieses Textes sind (karbonisierte) Getränke auf (Mineral-) Wasserbasis, die meist klar sind, nur schwach gefärbt werden, oft nur schwach gesüßt sind (weniger als 5 % Sucrose oder Süßstoffe mit einer Süßkraft von weniger als 5 % Sucrose), meist gar nicht oder nur schwach angesäuert sind und dabei einen pH-Bereich von ca. 4 bis 8 umfassen, meist nur aromatisiert sind und dabei noch mit Mineralien, Vitaminen und/oder Pflanzenextrakten versehen werden können. Im Gegensatz zu den meisten anderen Getränken (z.B. Limonaden, Fruchtsaftgetränken, [Eis-]Teegetränken u.s.w.) steht dabei der "Wasser"-Charakter des Getränks immer noch im Vordergrund.

Bevorzugt sind dabei die Getränke, die einen pH-Wert kleiner 7, besonders bevorzugt kleiner 5, insbesondere bevorzugt kleiner 4 haben.

### F. Mund- und Zahnpflegemittel

Erfindungsgemäß zu verwendende, oral konsumierbare süß schmeckende Produkte können auch der Mund- und Zahnreinigung und-pflege dienen. Beispiele hierfür sind Zahnpasten, Zahngele, Zahnpulver, Mundwässer und dergleichen. Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete **Putzkörper** für die erfindungsgemäßen Zahnpasten sind vor allem feinteilige Xerogelkieselsäuren, Hydrogelkieselsäuren, Fällungskieselsäuren, Aluminiumoxidtrihydrat und feinteiliges alpha -Aluminiumoxid oder Mischungen dieser Putzkörper in Mengen von 15 bis 40 Gew.-% der Zahnpasta.

Als **Feuchthaltemittel** kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol®-Typen) geeignet.

Zusätzlich können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süßungsmittel, wie z. B. Saccharin-Natrium, Natrium-Cyclamat, Sucrose, Lactose, Maltose, Fructose oder Apartam®, (L-Aspartyl- L-phenylalanin-methylester), Stivia-extrakte oder deren süßenden Bestandteile, insbesondere Ribeaudioside;
- geschmacksmodulierende Stoffe (beispielsweise Inositolphosphat, Nucleotide wie Guanosinmonophosphat, Adenosinmonophosphat oder andere Stoffe wie Natriumglutamat oder 2-Phenoxypropionsäure),
- Kühlwirkstoffen wie beispielsweise Menthol, Mentholderivate (beispielsweise L-Menthol, L-Menthyllactat, L-Menthylalkylcarbonate, Menthonketale, Menthancarbonsäureamide), 2,2,2-Trialkylessigsäureamiden (beispielsweise 2,2-Diisopropylpropionsäuremethylamid), Icilin-Derivate,
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner **Hydrotrope,** wie beispielsweise Ethanol, Isopropylalkohol, oder Polyole eingesetzt werden; diese Stoffe entsprechen weitgehend den eingangs geschilderten Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### G. Kaugummis

Bei den bevorzugten oralen Zubereitungen kann es sich auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang lkang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süßstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### H. Pharmazeutische Zubereitungen

Sofern die oralen Zubereitungen pharmazeutische Mittel darstellen, umfassen diese einen pharmazeutischen Wirkstoff. Vorteilhafte pharmazeutische Wirkstoffe sind beispielsweise steroidale entzündungshemmende Substanzen vom Kortikosteroiden-Typ wie beispielsweise Hydrocortison, Hydrocortison-Derivate wie Hydrocortison-17-butyrat, Dexamethason, Dexamethasonphosphat, Methylprednisolon oder Cortison.

Vorteilhafte nichtsteroidale pharmazeutische Wirkstoffe sind beispielsweise Entzündungshemmer wie Oxicame wie Piroxicam oder Tenoxicam; Salicylate wie Aspirin® (Acetylsalicylsäure), Disalcid, Solprin oder Fendosal; Essigsäure-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Flurbiprofen, Benoxaprofen oder Pyrazole wie Phenylbutazon, Oxyphenylbutazon, Febrazon oder Azapropazon.

Besonders bevorzugte pharmazeutische Zubereitungen sind nicht verschreibungspflichtige Produkte und frei verkäufliche Arzneimittel, sogenannte OTC ("over the counter") - Präparate, enthaltend Wirkstoffe wie Paracetamol, Acetylsalicylsäure oder Ibuprofen, Vitamine (beispielsweise Vitamin H, Vitamine aus der B-Reihe wie Vitamin B1, B2, B6, B12, Niacin, Panthotensäure, vorzugsweise in Form von (Brause)Tabletten oder Kapseln), Mineralien (vorzugsweise in Form von (Brause)Tabletten oder Kapseln) wie Eisensalze, Zinksalze, Selensalze, Produkte enthaltend Wirkstoffe oder Extrakte von Spitzwegerich (beispielsweise in Hustensirup) oder Johanniskraut.

### I. Kapseln

Die oralen Zubereitungen können auch in Emulsionen, in Liposomen, beispielsweise ausgehend von Phosphatidylcholin, in Microsphären, in Nanosphären und insbesondere auch in Kapseln, Granulaten oder Extrudaten aus einer für Lebens- und Genussmittel geeigneten Matrix, beispielsweise aus Stärke, Stärkederivaten, Cellulose oder Cellulosederivaten (beispielsweise Hydroxypropylcellulose) eingearbeitet werden.

In eine weiteren bevorzugten Ausführungsform werden die erfindungsgemäßen Aromamischungen mit einem oder mehreren geeigneten Komplexbildnern, beispielsweise mit Cycloglycanen, beispielsweise Cyclofructanen, Cyclodextrinen oder Cyclodextrinderivaten, bevorzugt α-, γ- und ?-Cyclodextrin, komplexiert und in dieser komplexierten Form als konfektioniertes Endprodukt, d.h. als orale Zubereitung eingesetzt. Besonders bevorzugt ist eine erfindungsgemäßes oral konsumierbares süß schmeckendes Produkt, bei der die Matrix so gewählt wird, dass die erfindungsgemäße Aromamischung verzögert von der Matrix freigegeben wird, so dass man eine langanhaltende Wirkung erhält.

Neben üblichen Makrokapseln auf Basis von Gelatine kommen dabei vor allem auch so genannte Mikro- oder Nanokapseln in Betracht. Darunter werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon. Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide). Besonders interessant für die Verkapselung von Zubereitungen für kosmetische Anwendungen sind Koazervate von kationischen Polymeren, insbesondere von Chitosan, mit anioniscchen polymeren, speziell Alginaten. Entsprechende Verfahren sind beispielsweise in den Druckschriften WO 2001 001926**,** WO 2001 001927**,** WO 2001 001928 **und** WO 2001 001929 (Cognis) beschrieben.

Mikrokapseln enthalten die Wirkstoffe häufig in einer Gelphase gelöst oder dispergiert. Als Gelbildner werden vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (La-mequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol. Vorzugsweise wird als Verkapselungsmaterial Chitosan eingesetzt. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

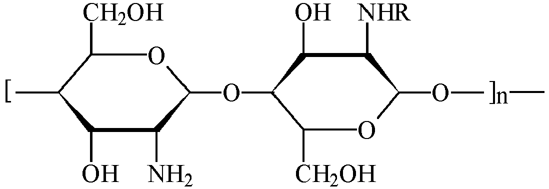

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

Die anionischen Polymere haben die Aufgabe, mit den kationischen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

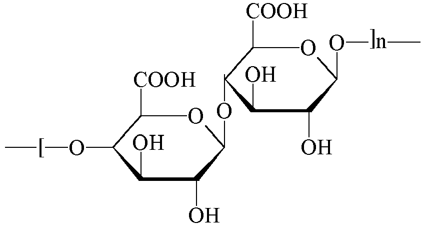

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100 °C, wird eine zweite wässrige Lösung zugegeben, welche das Kationpolymer, vorzugsweise das Chitosan in Mengen von 0,1 bis 2, vorzugsweise 0,25 bis 0,5 Gew.-% und den Wirkstoffen in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Kationpolymer und Wirkstoffen kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Kationpolymers in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 0,01 bis 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Kationpolymeren, speziell den Chitosanen durchführen.

Alternativ kann die Verkapselung auch unter ausschließlicher Verwendung von Kationpolymeren erfolgen, wobei man sich deren Eigenschaft zu Nutze macht, bei pH-Werten oberhalb des pKs-Wertes zu koagulieren.

In einem zweiten alternativen Verfahren wird zur Herstellung der erfindungsgemäßen Mikrokapseln wird zunächst eine O/W-Emulsion zubereitet, welche neben dem Ölkörper, Wasser und den Wirkstoffen eine wirksame Menge Emulgator enthält. Zur Herstellung der Matrix wird diese Zubereitung unter starkem Rühren mit einer entsprechenden Menge einer wässrigen Anionpolymerlösung versetzt. Die Membranbildung erfolgt durch Zugabe der Chitosanlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diestern von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.

In einem dritten alternativen Verfahren erfolgt die Bildung der Mikrokapseln um einen vorzugsweise festen, beispielsweise kristallinen Kern, indem dieser schichtweise mit entgegengesetzt geladenen Polyelektrolyten eingehüllt wird. In diesem Zusammenhang sei auf das Europäische Patent EP 1064088 B1 (Max-Planck Gesellschaft) verwiesen.

### Gewerbliche Anwendbarkeit

Bei der Verwendung der besagten Milchsäure-l-menthylester zur Maskierung von unangenehmen Geschmackseindrücken bei Zubereitungen zur oralen Aufnahme können die Einsatzmengen im Bereich von deutlich unter 5 mg/kg, vorzugsweise unter 2 mg/kg und vorzugsweise etwa 0,005 bis etwa 0,1 mg/kg - jeweils bezogen auf die Gesamtzubereitung - betragen.

### Beispiele

### Beispiel 1

### Minderung der Adstringenz über die Zeit von Epigallocatechingallat

Um verschiedene Attribute einer Probe gleichzeitig über einen bestimmten Zeitraum zu bewerten, wurde die Methode das so genannte "Multiple Time-Intensity Profilng" (mTIP) herangezogen. Diese deskriptive Methode vereinigt die Vorteile der "Dual-Attribute Time-Intensity" (DATI) -Methode, bei der die Intensitäten von 2 Deskriptoren auf einer zweidimensionalen Ebene aufgetragen werden können, sowie der "Time-Intensity Profiling" (TIP) -Methode, bei der mehr als zwei Deskriptoren nacheinander über die Zeit evaluiert werden.

Bei jedem mTIP-Test werden trainierten Panelisten (n=15) gebeten, nacheinander 2 Schlucke (je 5 ml) der zu bewertenden Probe in den Mund zu nehmen, den kompletten Mundraum damit zu benetzen und die Probe danach wieder auszuspucken. Die Messung beginnt, indem die Intensitäten mehrerer Deskriptoren auf horizontalen Linienskalen, die auf dem Computerbildschirm abgebildet sind, markiert werden. Für jeden Deskriptor ist jeweils eine Linienskala abgebildet. Somit ist eine parallele Bewertung von mehr als zwei Attributen möglich. Die Intensitäten werden nach den ersten 10 Sekunden und im Anschluss daran alle 20 Sekunden über einen Zeitraum von 70 Sekunden abgefragt. Die dabei entstehenden Daten werden mittels Compusense® *five* analysiert und grafisch in einer Zeit-Intensitäts-Kurve dargestellt.

Um den adstringenz-maskierenden Effekt von L-Milchsäure-L-menthylester (Frescolat ML) aufzuzeigen wurde das Panel unter anderem mit einer wässrigen Testlösung geschult, die 750 mg/kg Epigallocathechingallat und 125 mg/kg Ascorbinsäure enthielt. Die Panelisten wurden gebeten, die Testlösung mit der oben beschriebenen Methode in Bezug auf ihre Bitterkeit und Adstringenz hin zu bewerten. In einem anschließenden Test folgte die Bewertung dieser beiden Deskriptoren zum einen von der oben beschriebenen Testlösung und zum anderen der Testlösung plus 300 µg/kg Frescolat ML. Die Ergebnisse beider Zeit-Intensitäts-Kurven wurden statistisch ausgewertet und miteinander verglichen, um einen adstringenz-maskierenden Effekt von Frescolat® ML zu bestimmen. Der Test erfolgte in Dreifachbestimmung und die Mittelwerte der Adstringenz-Reduktion sind in Tabelle 1 abgebildet. Für die Signifikanzberechung wurde der Student t-Test, doppelt mit gepaarten Stichproben, herangezogen.

**Tabelle 1**

| Adstringenz-Reduktion von 750 mg/kg EGCG (+ 125 mg/kg Ascorbinsäure) nach Zugabe von 300 µg/kg von L-Milchsäure-L-menthylester (Frescolat® ML), Mittelwert aus 2 Wiederholungen | | | |
|---|---|---|---|
| **Zeit (Sek.)** | **Adstringenz ohne Frescolat ML** | **Adstringenz mit Frescolat ML** | **Reduktion (%)** |
| 0 | 4,84 | 4,81 | -0,49 |
| 10 | 4,80 | 4,11 | -14,29 |
| 30 | 4,26 | 3,38 | -20,58* |
| 50 | 3,32 | 2,58 | -22,24* |
| 70 | 2,49 | 2,04 | -17,82 |

| | | | |
|---|---|---|---|
| *signifikant (p < 0.1) | | | |

### Anwendungsbeispiel 1

In **Tabelle 2** sind verschiedene flüssige Aromakompositionen angegeben. Die Stoffe bzw. Lösungen wurden in den unten angegebenen Mengenverhältnissen gemischt und dann mit Propylenglykol oder Ethanol aufgenommen, durch leichtes Erwärmen vollständig gelöst und homogen gemischt.

**Tabelle 2**

| Formulierungsbeispiele | | | | | | | | |
|---|---|---|---|---|---|---|---|---|
| **Inhaltsstoff** | **Zubereitung (Einsatz in Gew.-%)** | | | | | | | |
| | **A** | **B** | **C** | **D** | **E** | **F** | **G** | **H** |
| Frescolat® ML | 1 | 0,6 | 1 | 0,55 | 1,95 | 1 | 0,25 | 1,6 |
| Hesperetin | 2 | - | - | 2 | - | - | - | - |
| Symrise Zucker Aroma in Propylenglykol-1,2 | 1,5 | 4 | - | - | 2 | - | 0,3 | - |
| Symrise Milch Aroma in Propylenglykol-1,2 | - | - | - | 4 | - | - | - | - |
| Symrise Sahne Aroma in Propylenglycol-1,2 | - | - | 3 | 0,3 | 0,5 | 5 | 1 | 2,5 |
| Propylenglykol-1,2 | ad 100 | - | ad 100 | ad 100 | ad 100 | ad 100 | - | ad 100 |
| Ethanol | - | ad 100 | - | - | - | - | ad 100 | - |

### Anwendungsbeispiel 2

In **Tabelle 3** sind verschiedene trockene Aromakompositionen angegeben. Die Stoffe wurden in den unten angegebenen Mengenverhältnissen gemischt und mit den festen Trägerstoffen homogen gemischt

**Tabelle 3**

| Formulierungsbeispiele | | | | | | | | |
|---|---|---|---|---|---|---|---|---|
| **Inhaltsstoff** | **Zubereitung (Einsatz in Gew.-%)** | | | | | | | |
| | **A** | **B** | **C** | **D** | **E** | **F** | **G** | **H** |
| Frescolat® ML | 25 | 15 | 0,5 | 1 | 1 | 0,25 | 1,2 | 6 |
| Ca. 10 Gew.% Pellitorin in Lösung Propylenglykol-1,2 | - | - | - | - | - | - | - | 10 |
| Ca. 10 Gew-% Homoeriodictiol sprühgetrocknet | - | - | 35 | 50 | 45 | 15 | 13 | 20 |
| Ca. 10 Gew.-% Hesperetin sprühgetrocknet | - | - | - | - | 10 | 10 | 6 | - |
| Symrise Abrundungsaroma für Tee sprühgetrocknet | - | - | 1 | - | 30 | 5 | - | - |
| Symrise Zucker Aroma in Triacetin | 8 | 5 | 0,5 | - | - | - | - | - |
| Symrise Vanille sprühgetrocknet | - | - | - | - | - | - | 3 | 10 |
| Dextrose wasserfrei | - | ad 100 | - | - | - | ad 100 | - | - |
| Maltodextrin | ad 100 | - | ad 100 | ad 100 | ad 100 | - | ad 100 | ad 100 |

### Anwendungsbeispiel 3

In **Tabelle 4** sind verschiedene sprühgetrocknete Aromakompositionen als Halbfertigwaren zur Aromatisierung von Fertigwaren angegeben. Das Trinkwasser wurde in einem Behälter vorgelegt und das Maltodextrin und das Gummi Arabicum darin gelöst. Anschließend wurden Bestandteile mit einem Mixer (Turrax) in die unten beschriebene Trägerstofflösung emulgiert. Die Temperatur des resultierenden Gemisches sollte 30°C nicht überschreiten. Das Gemisch wurde dann sprühgetrocknet (Solltemperatur Eingang: 185 - 195°C, Solltemperatur Ausgang: 70 - 75°C). Analog können auch sprühgetrocknete Zubereitungen mit erfindungsgemäßen anderen Aromakompositionen hergestellt werden.

**Tabelle 4**

| Formulierungsbeispiele | | | | | | |
|---|---|---|---|---|---|---|
| **Inhaltsstoff** | **Zubereitung (Einsatz in Gew.-%)** | | | | | |
| | **A** | **B** | **C** | **D** | **E** | **F** |
| Maltodextrin aus Weizen | 24,3 | 24,3 | 24,3 | 24,3 | 24,3 | 24,3 |
| Gummi Arabicum | 6,1 | 6,1 | 6,1 | 6,1 | 6,1 | 6,1 |
| Frescolat® ML | 0,1 | 0,08 | 0,04 | 0,06 | 0,1 | 0,1 |
| Symrise Zucker Aroma in Triacetin | - | 0,5 | 0,2 | - | 0,1 | - |
| Symrise Sahne Aroma in Triacetin | - | - | - | 0,05 | 0,05 | - |
| Symrise Milch Aroma in Triacetin | - | - | - | - | - | 0,1 |
| Trinkwasser | ad 100 | ad 100 | ad 100 | ad 100 | ad 100 | ad 100 |

### Anwendungsbeispiel 4

In Tabelle 5 sind Aromamischungen zur Reduktion der unangenehmen Geschmackseindrücke in einer Süßstoffmischung zum Süßen von Kakao-, Kaffee- oder Tee-haltigen Getränken angegeben. Die Zubereitungen A bis D wurden mit 1% auf schwarzen Kaffee (frisch gebrüht) dosiert. Die Zubereitungen B, C und D sind erfindungsgemäß, A dient zum Vergleich. Verglichen mit Zubereitung A (Vergleich) war in Zubereitung B - D die Adstringenz und die langanhaltende Süße vermindert. Die metallischen Noten wurden zudem reduziert. Zubereitung D hatte zudem einen Sucrose-typischeren Geschmack.

**Tabelle 5**

| Formulierungsbeispiele | | | | |
|---|---|---|---|---|
| **Inhaltsstoff** | **Zubereitung (Angaben als Gew.-%)** | | | |
| | **A** | **B** | **C** | **D** |
| Saccharin | 1,50 | 1,50 | 1,50 | 1,50 |
| Aromakomposition nach Anwendungs-beispiel 2 B | - | 1,7 | - | 0,6 |
| Aromakomposition nach Anwendungs-beispiel 2 A | - | - | - | 1,00 |
| Aromakomposition nach Anwendungsbeispiel 3 B | - | - | 1,00 | - |
| Sorbitol | ad 100 | ad 100 | ad 100 | ad 100 |

### Anwendungsbeispiel 5

In **Tabelle 6** sind Beispiele für die Verwendung von Milchsäure-l-menthylester in einer Süßstoffmischung zum Süßen von fettreduzierten, fettarmen bzw. fettfreien Milchprodukten zur Reduktion der unangenehmen Geschmackseindrücke angegeben. Die Zubereitungen A bis D wurden mit 1% auf schwarzen Kaffee (frisch gebrüht) dosiert. Die Zubereitungen B, C und D sind erfindungsgemäß, A dient zum Vergleich. Die Aromakompositionen sowie die Süßstoffe wurden mit neutralem 0,1 % Fett-haltigen Joghurt verrührt. Die Mischungen erfordern eine Reifezeit von 3 Tagen. Verglichen mit Zubereitung A (Vergleich) war in Zubereitung B - D die Adstringenz und die langanhaltende Süße vermindert. Die metallischen Noten wurden zudem reduziert. In Zubereitung C wurde eine deutlich stärkere Mundfülle erreicht (an Sucrose erinnernd).

**Tabelle 6**

| Formulierungsbeispiele | | | | |
|---|---|---|---|---|
| **Inhaltsstoff** | **Zubereitung (Angaben als Gew.-%)** | | | |
| | **A** | **B** | **C** | **D** |
| Aromakomposition nach Anwendungsbeispiel 1 E | - | 0,03 | - | - |
| Aromakomposition nach Anwendungsbeispiel 1 A | - | - | - | 0,08 |
| Aromakomposition nach Anwendungs-beispiel 1 H | - | - | 0,03 | - |
| Acesulfam K | 0,01 | 0,01 | 0,01 | 0,01 |
| Aspartam | 0,02 | 0,02 | 0,02 | 0,02 |
| Joghurt natur, 0,1% Fett | ad 100 | ad 100 | ad 100 | ad 100 |

### Anwendungsbeispiel 6

In **Tabelle 7** sind Beispiele für die Verwendung von Milchsäure-l-menthylester in einem Sojamilch-Getränk angegeben. Die Zubereitungen B bis H sind erfindungsgemäß, A dient zum Vergleich. Die Aromakompositionen wurden mit der neutralen Sojamilch gemischt. Die Mischungen erforderten eine Reifezeit von 5 bis 6 Tagen. Verglichen mit Zubereitung A (Vergleich) war in Zubereitung B bis H die Adstringenz der Sojamilch vermindert. In Zubereitung B bis D, sowie G+H wurde die Mundfülle der Sojamilch deutlich verbessert, die Soja-bohnigen Noten wurden reduziert. In Zubereitung E und F wurde neben der Adstringenz besonders die Bitterkeit der Sojamilch reduziert.

**Tabelle 7**

| Formulierungsbeispiele | | | | | | | | |
|---|---|---|---|---|---|---|---|---|
| **Inhaltsstoff** | **Zubereitung (Angaben als Gew.-%)** | | | | | | | |
| | **A** | **B** | **C** | **D** | **E** | **F** | **G** | **H** |
| Aromakomposition nach Anwendungsbeispiel 3A | 0,015 | - | - | - | - | - | - | - |
| Aromakomposition nach Anwendungsbeispiel 1 G | - | - | 0,1 | - | - | - | - | - |
| Aromakomposition nach Anwendungsbeispiel 1 F | - | 0,04 | - | - | - | - | - | - |
| Aromakomposition nach Anwendungsbeispiel 1 C | - | - | - | 0,01 | - | - | 0,005 | - |
| Aromakomposition nach Anwendungsbeispiel 1 A | - | - | - | - | - | 0,1 | - | - |
| Aromakomposition nach Anwendungsbeispiel 1 D | - | - | - | - | 0,15 | - | 0,1 | - |
| Aromakomposition nach Anwendungsbeispiel 3 E | - | - | - | - | - | - | - | 0,015 |
| Sojamilch, ohne Zusätze (lokaler Supermarkt) | ad 100 | ad 100 | ad 100 | ad 100 | ad 100 | ad 100 | ad 100 | ad 100 |

### Anwendungsbeispiel 7

In **Tabelle 8** ist ein Beispiel für die Verwendung von Milchsäure-l-menthylester in einem Soja-Getränk in Kombination mit γ-Aminobuttersäure. γ-Aminobuttersäure wurde in Wasser vorgelöst und zusammen mit einer erfindungsgemäßen Aromamischung zu einer Sojamilch aus einem lokalen Supermarkt hinzugefügt. Die Mischung wurde zusammen mit dem Milcharoma im Becherglas verrührt. Im Vergleich zu Anwendungsbeispiel 6 B wurde die Adstringenz deutlich reduziert, die Sojamilch erhält eine sehr neutrale Frische.

**Tabelle 8**

| Formulierungsbeispiel | |
|---|---|
| **Inhaltsstoff** | **Einsatz in Gew.-%** |
| Sojamilch, ohne Zusätze (lokaler Supermarkt) | 99,76 |
| Aromakomposition nach Anwendungsbeispiel 1 F | 0,04 |
| 1 % γ-Aminobuttersäure in Wasser | 0,2 |

### Anwendungsbeispiel 8

In **Tabelle 9** sind Beispiele für die Verwendung von Milchsäure-l-menthylester in einem Soja-Frucht-Getränk angegeben. Die Zubereitungen B bis D sind erfindungsgemäß, A dient zum Vergleich. Die Aromakompositionen wurden mit den restlichen Zutaten gemischt. Die Mischung wurde homogenisiert und anschließend pasteurisiert (15 Min. bei 80-85°C). Verglichen mit Zubereitung A (Vergleich) waren in Zubereitung B bis D die Adstringenz und die Soja-bohnigen Noten vermindert. In Zubereitung B wurde zudem die Bitterkeit deutlich reduziert. Alle Proben hatten einen deutlich fruchtigeren Angeschmack.

**Tabelle 9**

| Formulierungsbeispiele | | | | |
|---|---|---|---|---|
| **Inhaltsstoff** | **Zubereitung (Angaben als Gew.-%)** | | | |
| | **A** | **B** | **C** | **D** |
| Aromakomposition nach Anwendungsbeispiel 1 D | - | 0,12 | 0,1 | - |
| Aromakomposition nach Anwendungsbeispiel 1 A | - | - | - | 0,08 |
| Aromakomposition nach Anwendungsbeispiel 1 C | - | - | 0,003 | - |
| Süßstoffmix | 0,03 | 0,03 | 0,03 | 0,03 |
| Zucker | 5 | 5 | 5 | 5 |
| Fruchtsaftmix aus Fruchtsaftkonzentraten | 50 | 50 | 50 | 50 |
| Wasser | ad 100 | ad 100 | ad 100 | ad 100 |
| Soja pulver | 5 | 5 | 5 | 5 |

### Anwendungsbeispiel 9

In **Tabelle 10** sind Beispiele für die Verwendung von Milchsäure-l-menthylester in einer Sojaeiscreme angegeben. Die Zubereitungen B bis D sind erfindungsgemäß, A dient zum Vergleich. Die Aromakompositionen werden mit dem Eiscrememix gemischt. Anschließend wird die Masse im Freezer mit einem Aufschlag von 100% gefroren und bei -25°C gelagert. Verglichen mit Zubereitung A (Vergleich) waren in Zubereitung B bis D die Soja-bohnigen Noten deutlich reduziert.

**Tabelle 10**

| Formulierungsbeispiele | | | | |
|---|---|---|---|---|
| **Inhaltsstoff** | **Zubereitung (Angaben als Gew.-%)** | | | |
| | **A** | **B** | **C** | **D** |
| Aromakomposition nach Anwendungsbeispiel 1 F | - | 0,06 | - | - |
| Aromakomposition nach Anwendungsbeispiel 1 G | - | - | - | 0,12 |
| Aromakomposition nach Anwendungsbeispiel 1 C | - | - | 0,014 | - |
| Sojaeiscreme-Mix (mit 12% Saccharos, 8% Glucofructose Sirup, 3% Sojapulver, 4,5% Fett) | ad 100 | ad 100 | ad 100 | ad 100 |

### Anwendungsbeispiel 10

In **Tabelle 11** sind Beispiele für die Verwendung von Milchsäure-l-menthylester in einem fettarmen Joghurt angegeben. Die Zubereitungen B bis D sind erfindungsgemäß, A dient zum Vergleich. Die Aromakompositionen sowie die Sucrose werden mit dem neutralen 0,1% Fett-haltigen Joghurt verrührt. Die Mischungen erfordern eine Reifezeit von 3 Tagen. Verglichen mit Zubereitung A (Vergleich) war in Zubereitung B - D die Adstringenz vermindert. Zudem wurde die Säure des Joghurts vermindert. In Zubereitung D wurde eine deutlich stärkere Mundfülle erreicht.

**Tabelle 11**

| Formulierungsbeispiele | | | | |
|---|---|---|---|---|
| **Inhaltsstoff** | **Zubereitung (Angaben als Gew.-%)** | | | |
| | **A** | **B** | **C** | **D** |
| Aromakomposition nach Anwendungsbeispiel 1 F | - | 0,03 | - | - |
| Aromakomposition nach Anwendungsbeispiel 1 G | - | - | - | 0,1 |
| Aromakomposition nach Anwendungsbeispiel 3 F | - | - | 0,015 | - |
| Sucrose | 5 | 5 | 5 | 5 |
| Joghurt natur, 0,1% Fett | ad 100 | ad 100 | ad 100 | ad 100 |

### Anwendungsbeispiel 11

In **Tabelle 12** ist ein Beispiel für die Verwendung von Milchsäure-l-menthylester in einer fettreduzierten Mayonnaise angegeben. Im Vergleich zu fettreduzierter Mayonnaise ohne Aromakomposition wurde die Adstringenz verbessert, die Zubereitung erhielt ein besseres Mundgefühl/Fettigkeit.

**Tabelle 12**

| Formulierungsbeispiel | |
|---|---|
| **Inhaltsstoff** | **Einsatz in Gew.-%** |
| Fettreduzierte Mayonnaise | 99,93 |
| Aromakomposition nach Anwendungsbeispiel 1 F | 0,07 |

### Anwendungsbeispiel 12

In **Tabelle 13** sind Beispiele für die Verwendung von Milchsäure-l-menthylester in einer Grüntee-Zubereitung angegeben. Der Grünteextrakt, Säure und Süßstoffmischung sowie die Aromakompositionen wurden in 80° heißem Wasser gelöst und in Flaschen abgefüllt. Im Vergleich zur Kontroll-Grüntee-Zubereitung (ohne Aromakomposition) wurde die Adstringenz reduziert. Die Bitterkeit war zudem besonders in Zubereitung B reduziert; Zubereitung B zeigte eine deutlich vollere Süße.

**Tabelle 13**

| Formulierungsbeispiele | | |
|---|---|---|
| **Inhaltsstoff** | **Einsatz in Gew.-%** | |
| | **A** | **B** |
| Grüntee-Extrakt, ca. 16% Catechingehalt | 0,25 | 0,25 |
| Aromakomposition nach Anwendungsbeispiel 2 D | 0,1 | - |
| Aromakomposition nach Anwendungsbeispiel 2 E | - | 0,1 |
| Süßstoffmischung (Aspartam, Sucralose 1:1) | 0,01 | 0,01 |
| Äpfelsäure, Citronensäure | 0,1 | 0,1 |
| Demineralisiertes Wasser | ad 100 | ad 100 |

### Anwendungsbeispiel 13

In **Tabelle 14** sind Beispiele für die Verwendung von Milchsäure-l-menthylester in verschiedenen Tee-Zubereitungen angegeben. Teeblätter und die Halbfertigprodukte wurden gemischt und in Filterbeutel bepackt. Für die Verkostung wurden 100 bis 250 ml kochendes Wasser auf den Teebeutel gegeben und 2 bis 5 min ziehen gelassen.

**Tabelle 14**

| Formulierungsbeispiele | | | |
|---|---|---|---|
| **Inhaltsstoff** | **Einsatz in Gew.-%** | | |
| | A | B | C |
| Schwarztee, Ceylon, Blätter | 94 | - | - |
| Grüntee, China, Blätter | - | 92 | - |
| Mate Tee, Peru, Blätter | - | - | 95 |
| Halbfertigprodukt A aus Beispiel 3 | 6 | - | - |
| Halbfertigprodukt B aus Beispiel 3 | - | 8 | - |
| Halbfertigprodukt C aus Beispiel 3 | - | - | 5 |

### Anwendungsbeispiel 14

In **Tabelle 15** sind Beispiele für die Verwendung von Milchsäure-l-menthylester in weiteren Tee-Zubereitungen angegeben. Teeblätter und die Halbfertigprodukte wurden gemischt und in Filterbeutel bepackt. Für die Verkostung werden 100 bis 250 ml kochendes Wasser auf den Teebeutel gegeben 2 bis 5 min ziehen gelassen.

**Tabelle 15**

| Formulierungsbeispiele | | |
|---|---|---|
| **Inhaltsstoff** | **Einsatz in Gew.-%** | |
| | **A** | **B** |
| Schwarztee, Ceylon, Blätter | 94 | - |
| Grüntee, Japan Sencha, Blätter | - | 95 |
| Halbfertigprodukt A aus Beispiel 2 | 3 | - |
| Halbfertigprodukt C aus Beispiel 2 | 3 | 5 |

### Anwendungsbeispiel 15

In **Tabelle 16** sind Beispiele für die Verwendung von Milchsäure-l-menthylester in Eistee-Zubereitungen auf Basis von Schwarztee angegeben.

**Tabelle 16**

| Formulierungsbeispiele | | |
|---|---|---|
| **Inhaltsstoff** | **Einsatz in Gew.-%** | |
| | **A** | **B** |
| Schwarztee, Assam, Blätter | 1.4 | 1.4 |
| Wasser | ad 100 | ad 100 |
| Natürliches Pfirsicharoma | 0.65 | 0.65 |
| Sucrose | 7 | 7 |
| Zitronensäure (kristallin) | 1.2 | 1.2 |
| Ascorbinsäure | 0.2 | 0.2 |
| Frescolat® ML | 0.00003 | - |
| Homoeriodictyol (10% in Ethanol) | 0,01 | - |
| Matairesinol (10% in Ethanol) | - | 0,01 |

### Anwendungsbeispiel 16

In **Tabelle 17** sind Beispiele für die Verwendung von Milchsäure-l-menthylester in kalorienreduzierten Eistee-Zubereitungen auf Basis von Grüntee angegeben.

**Tabelle 17**

| Formulierungsbeispiele | | |
|---|---|---|
| **Inhaltsstoff** | **Einsatz in Gew.-%** | |
| | **A** | **B** |
| Grüntee Extrakt, Catechingehalt 10 Gew.% | 1.4 | 1.2 |
| Wasser | ad 100 | ad 100 |
| Aroma Typ Zitrone | 0.65 | 0.65 |
| Sucrose | 3.45 | 3.45 |
| Saccharin, Natriumsalz | 0.1 | - |
| Rebaudiosid A | - | 0.02 |
| Zitronensäure (kristallin) | 1.2 | 1.2 |
| Ascorbinsäure | 0.2 | 0.2 |
| Frescolat® ML | 0.00005 | 0.00003 |

### Anwendungsbeispiel 17

In **Tabelle 18** sind Beispiele für die Verwendung von Milchsäure-l-menthylester in kalorienreduzierten Eistee-Zubereitungen auf Basis von Schwarztee angegeben.

**Tabelle 18**

| Formulierungsbeispiele | | |
|---|---|---|
| **Inhaltsstoff** | **Einsatz in Gew.-%** | |
| | **A** | **B** |
| Schwarztee Extrakt | 1.4 | 1.4 |
| Wasser | Ad 100 | Ad 100 |
| Saccharin | 0.035 | 0.035 |
| Aroma Typ Zitrone | 0.65 | 0.65 |
| Zitronensäure (kristallin) | 1.2 | 1.2 |
| Ascorbinsäure | 0.2 | 0.2 |
| Frescolat® ML | 0.00005 | 0,00004 |
| Eriodictyol (10% in Ethanol) | - | 0.05 |
| Schwarztee Extrakt | 1.4 | 1.4 |

### Anwendungsbeispiel 18

In **Tabelle 19** ist ein Beispiel für die Verwendung von Milchsäure-l-menthylester in einer bitteren Schokolade angegeben. Die Zutaten wurden in die bei 40 °C geschmolzene Schokolade eingearbeitet und die flüssige Masse in Tafelform gegossen und nach dem dem Fachmann bekannten Temperungsverfahren abgekühlt, wobei man eine Essschokolade erhielt. Die so zubereitete Schokolade wurde von den trainierten Fachleuten als weniger bitter, weniger adstringierend und insgesamt als mehr abgerundet bezeichnet.

**Tabelle 19**

| Formulierungsbeispiel | |
|---|---|
| **Inhaltsstoff** | **Einsatz in Gew.-%** |
| Bitterschokolade, min. 85 % Kakao (Handelsprodukt) | 99,7 |
| Aromakomposition nach Anwendungsbeispiel 2G | 0,3 |

## Patentansprüche

1. Verwendung von Milchsäure-1-menthylestern der Formel (I) zur Maskierung oder Reduzierung von unangenehmen Geschmackseindrücken bei Zubereitungen zur oralen Aufnahme 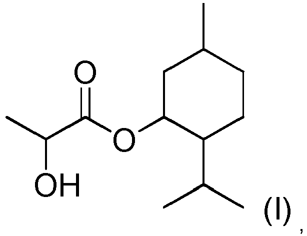 wobei die Konzentration der Milchsäure-1-menthylester der Formel (I) in den Zubereitungen zur oralen Aufnahme unter der Konzentration liegt, bei der die Verbindungen der Formel (I) einen wahrnehmbaren physiologischen Kühleffekt verursachen,
wobei es sich bei den unangenehmen Geschmackseindrücken um adstringierend, trocken, staubig, mehlig und/oder kalkig handelt,
und wobei die Zubereitungen zur oralen Aufnahme Stoffe umfassen, ausgewählt aus der Gruppe, die gebildet wird von
(b1) Catechinen,
(b2) Proanthocyanidinen,
(b3) Coffein,
(b4) Theobromin,
(b5) Naringin und
(b6) Süßstoffen.

2. Verwendung nach Anspruch 1, **dadurch gekennzeichnet, dass** man die Catechine und Proanthocyanidine in einer Gesamtmenge von 0,01 bis 1 Gew.-% - bezogen auf die oralen Zubereitungen - einsetzt.

3. Verwendung nach Anspruch 1, **dadurch gekennzeichnet, dass** man Coffein und Theobromin in einer Gesamtmenge von 0,005 bis 1 Gew.-% - bezogen auf die oralen Zubereitungen - einsetzt.

4. Verwendung nach Anspruch 1, **dadurch gekennzeichnet, dass** man Naringin in einer Menge von 0,005 bis 0,5 Gew.-% - bezogen auf die oralen Zubereitungen - einsetzt.

5. Verwendung nach Anspruch 1, **dadurch gekennzeichnet, dass** man die Süßstoffe in einer Gesamtmenge von 0,005 bis 2 Gew.-% einsetzt.

## Claims

1. Use of lactic acid 1-menthyl ester of formula (I) for masking or reducing unpleasant taste impressions in preparations for oral consumption 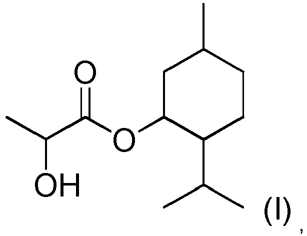 wherein the concentration of the lactic acid 1-menthyl ester of formula (i) in the preparations for oral consumption is below the concentration at which the compounds of formula (I) cause a perceptible physiological cooling effect,
wherein the unpleasant taste impressions are astringent, dry, dusty, mealy and/or limy,
and wherein the preparations for oral consumption comprise substances selected from the group consisting of
(b1) Catechins,
(b2) Proanthocyanidins,
(b3) Caffeine,
(b4) Theobromine,
(b5) Naringin and
(b6) Sweeteners.

2. Use of claim 1, **characterized in that** the catechins and the proanthocyanidins are used in a total amount of 0.01 to 1 % by weight - based on the oral preparations.

3. Use of claim 1, **characterized in that** the caffeine and the theobromine are used in a total amount of 0.005 to 1 % by weight, based on the oral preparations.

4. Use of claim 1, **characterized in that** the naringin is used in an amount of 0.005 to 0.5 % by weight, based on the oral preparations.

5. Use of claim 1, **characterized in that** the sweeteners are used in a total amount of 0.005 to 2 % by weight.

## Revendications

1. Utilisation d'esters 1-menthyliques d'acide lactique de formule (I) pour masquer ou réduire des impressions gustatives désagréables dans des préparations destinées à l'ingestion orale 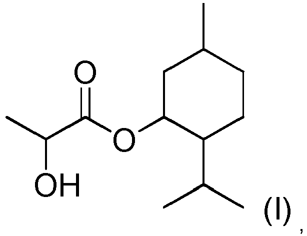 dans laquelle la concentration des esters 1-menthyliques d'acide lactique de formule (I) dans les préparations destinées à l'ingestion orale est inférieure à la concentration à laquelle les composés de formule (I) provoquent un effet de refroidissement physiologique perceptible,
dans laquelle les impressions gustatives désagréables sont astringentes, sèches, poussiéreuses, farineuses et/ou calcaires,
et dans laquelle les préparations destinées à l'ingestion orale comprennent des substances choisies dans le groupe constitué par
(b1) les catéchines,
(b2) les proanthocyanidines,
(b3) la caféine,
(b4) la théobromine,
(b5) la naringine et
(b6) les édulcorants.

2. Utilisation selon la revendication 1, **caractérisée par le fait que** les catéchines et les proanthocyanidines sont utilisées en une quantité totale comprise entre 0,01 et 1 % en poids, par rapport aux préparations orales.

3. Utilisation selon la revendication 1, **caractérisée par le fait que** la caféine et la théobromine sont utilisées en une quantité totale comprise entre 0,005 et 1 % en poids, par rapport aux préparations orales.

4. Utilisation selon la revendication 1, **caractérisée par le fait que** la naringine est utilisée en une quantité comprise entre 0,005 et 0,5 % en poids, par rapport aux préparations orales.

5. Utilisation selon la revendication 1, **caractérisée par le fait que** les édulcorants sont utilisés en une quantité totale comprise entre 0,005 et 2 % en poids.
